# EUROPEAN PATENT APPLICATION

(11) **EP 3 926 054 A1**
(43) Date of publication of application: **22.12.2021**
(21) Application number: 20756679.5
(22) Date of filing: 04.02.2020
(51) Int. Cl.: C12Q 1/689, C12Q 1/6883, C12Q 1/6886, C12Q 1/6851, A23L 33/135, A61K 35/74, A61P 1/16, A61P 3/10, A61P 9/00, A61P 17/00

(54) **NANOVESICLES DERIVED FROM BACTERIA OF GENUS ROTHIA, AND USE THEREOF**

(30) Priority: 14.02.2019 KR 20190017064; 03.02.2020 KR 20200012637
(71) Applicant: MD Healthcare Inc., Seoul 03923 (KR)
(72) Inventor: KIM, Yoon-Keun, Paju-Si, Gyeonggi-do 10908 (KR)
(74) Representative: Dehns
(86) International application number: PCT/KR2020/001612
(87) International publication number: WO 2020/166868

(57) **Abstract**

The present invention relates to vesicles derived from bacteria of the genus *Rothia* and a use thereof. The inventors experimentally confirmed that the vesicles significantly decreased in a clinical sample obtained from a patient with diabetes, atrial fibrillation, cardiomyopathy, liver cancer, cirrhosis, dementia, depression, Parkinson's disease, and atopic dermatitis, compared with a normal individual, and when the vesicles isolated from the strain were administered, the secretion of inflammation mediators caused by pathogenic vesicles such as *E. coli*-derived vesicles was considerably inhibited, and vesicles derived from bacteria of the genus *Rothia* significantly inhibit cranial nerve cell damage caused by stress hormones, and therefore, the vesicles derived from bacteria of the genus *Rothia* according to the present invention may be effectively used to develop a method of diagnosing diabetes, atrial fibrillation, cardiomyopathy, liver cancer, cirrhosis, dementia, depression, Parkinson's disease, and atopic dermatitis, and a composition for preventing or treating diabetes, cardiovascular diseases, liver disease, a cranial nerve disease, and an inflammatory disease.

## Description

### [Technical Field]

The present invention relates to nanovesicles derived from bacteria of the genus *Rothia* and a use thereof, and more particularly, to a method of diagnosing diabetes, atrial fibrillation, cardiomyopathy, liver cancer, cirrhosis, dementia, depression, Parkinson's disease, atopic dermatitis, or the like using nanovesicles derived from bacteria of the genus *Rothia,* and a composition for preventing, alleviating or treating diabetes, cardiovascular diseases, liver disease, a cranial nerve disease, and an inflammatory disease which comprises the vesicles.

This application claims priority to and the benefit of Korean Patent Application No. 10-2019-0017064, filed on February 14, 2019 and Korean Patent Application No. 10-2020-0012637, filed on February 3, 2020, and all the contents disclosed in the specification and drawings of the applications are incorporated in this application.

### [Background Art]

Since the beginning of the 21st century, acute infectious diseases recognized as epidemic diseases in the past have become less important, whereas chronic inflammatory diseases accompanied by immune dysfunction caused by disharmony between humans and microbiomes have changed disease patterns as main diseases that determine the quality of life and the human lifespan. Cancer, cardiovascular diseases, chronic pulmonary diseases, metabolic diseases and neuro-psychiatric diseases, which are intractable chronic diseases in the 21st century, are major diseases that determine human lifetimes and the quality of life, and become a big challenge to public health. The intractable chronic disease is characterized by chronic inflammation accompanied by abnormal immune function caused by a causative factor.

It is known that the number of microorganisms coexisting in the human body has reached 100 trillion, which is 10 times more than the number of human cells, and the number of microorganism genes is more than 100 times the number of human genes. A microbiota or microbiome refers to a microbial community including bacteria, archaea and eukarya present in a given habitat.

Meanwhile, bacteria coexisting in our body and bacteria present in the ambient environment secrete nanometer-sized vesicles in order to exchange information on genes, low molecular compounds, proteins, and the like with other cells. The mucosa forms a physical defense membrane through which particles having a size of 200 nanometers (nm) or more cannot pass, so that bacteria coexisting in the mucosa cannot pass through the mucosa, but vesicles derived from bacteria have a size of 100 nanometers or less and are absorbed into our bodies after relatively freely passing through epithelial cells via the mucosa. Bacteria-derived vesicles that are locally secreted from bacteria are absorbed via epithelial cells of the mucous membrane to thereby induce a local inflammatory response, and the vesicles having passed through the epithelial cells are systematically absorbed via lymphatic vessels and thereby distributed in respective organs, and immune and inflammatory responses are regulated in the organs in which the vesicles are distributed. For example, vesicles derived from pathogenic gram-negative bacteria such as *Escherichia coli* locally cause colitis, and promote a systemic inflammatory response, and blood coagulation through a vascular endothelial inflammatory response when absorbed into blood vessels, and cause insulin resistance and diabetes when absorbed into insulin-acting muscle cells and the like. On the other hand, vesicles derived from beneficial bacteria may control a disease by controlling immune dysfunction and metabolic dysfunction caused by pathogenic vesicles.

As immune responses to factors such as bacteria-derived vesicles, Th17 immune responses characterized by the secretion of the interleukin (hereinafter, IL)-17 cytokine occur, and IL-6 is secreted when exposed to bacteria-derived vesicles, thereby inducing Th17 immune responses. Inflammation caused by the Th17 immune response is characterized by neutrophil infiltration, and during the process by which inflammation occurs, tumor necrosis factor-alpha (hereinafter, TNF-α) secreted from inflammatory cells such as neutrocyte and macrophages plays an important role for occurrence of inflammation and cancer.

A brain-derived neurotrophic factor (BDNF) is a protein in the brain generated by BDNF gene, and one of a group of neurotrophic factors, as a part of the growth factors. The factor is associated with a basic nerve growth factor, and it is known that the expression of the factor is reduced in depression, dementia, Alzheimer's disease, autism, and the like.

Meanwhile, bacteria of the genus *Rothia* are anaerobic gram-positive bacteria coexisting in the oral cavity and the respiratory organs, and are known as bacteria generally not causing a disease. However, to date, the fact that vesicles are secreted extracellularly from bacteria of the genus *Rothia* has not been reported, and particularly, there have been no reported cases of application thereof in diagnosis and treatment of intractable diseases such as diabetes, atrial fibrillation, cardiomyopathy, liver cancer, cirrhosis, dementia, depression, Parkinson's disease and atopic dermatitis.

Accordingly, in the present invention, it was confirmed that vesicles derived from bacteria of the genus *Rothia* significantly decrease in clinical samples of diabetes, atrial fibrillation, cardiomyopathy, liver cancer, cirrhosis, dementia, depression, Parkinson's disease and atopic dermatitis patients, compared to a normal individual, thereby diagnosing the diseases. In addition, as a result of isolating vesicles from *Rothia amarae* belonging to the genus *Rothia* and evaluating the therapeutic effect thereof, it was confirmed that they can be used as a composition for preventing or treating diabetes, cardiovascular diseases, liver disease, a cranial nerve disease, an inflammatory disease, and the like.

### [Disclosure]

### [Technical Problem]

As a result of conducting earnest research to solve the above conventional problems, the inventors confirmed that a content of vesicles derived from bacteria of the genus *Rothia* is significantly decreased in a sample derived from a patient with diabetes, atrial fibrillation, cardiomyopathy, liver cancer, cirrhosis, dementia, depression, Parkinson's disease and atopic dermatitis patients, compared with a normal individual, through metagenomic analysis. In addition, when macrophages were treated with vesicles isolated from *Rothia amarae* belonging to the genus *Rothia,* it was confirmed that the secretion of IL-6 and TNF-alpha, which are inflammation mediators, caused by pathogenic vesicles was remarkably inhibited, and BDNF expression inhibiting cranial nerve cell damage caused by a stress hormone significantly increased, and therefore, based on this, the present invention was completed.

Thus, an object of the present invention is to provide a method of providing information for diagnosis of diabetes, atrial fibrillation, cardiomyopathy, liver cancer, cirrhosis, dementia, depression, Parkinson's disease, or atopic dermatitis.

Further, another object of the present invention is to provide a composition for preventing, alleviating, or treating one or more diseases selected from the group consisting of diabetes, cardiovascular diseases, liver disease, a cranial nerve disease, and an inflammatory disease, comprising vesicles derived from bacteria of the genus *Rothia* as an active ingredient.

However, a technical problem to be achieved by the present invention is not limited to the aforementioned problems, and the other problems that are not mentioned may be clearly understood by a person skilled in the art from the following description.

### [Technical Solution]

To achieve the object of the present invention as described above, the present invention provides a method of providing information for diagnosing diabetes, atrial fibrillation, cardiomyopathy, liver cancer, cirrhosis, dementia, depression, Parkinson's disease, or atopic dermatitis, the method comprising the following steps:
(a) extracting DNAs from vesicles isolated from samples of a normal individual and a subj ect;
(b) performing PCR (Polymerase Chain Reaction) on the extracted DNA using a pair of primers prepared based on a gene sequence present in 16S rDNA to obtain each PCR product; and
(c) classifying a case in which a content of vesicles derived from bacteria of the genus *Rothia* is lower than that of the normal individual sample, as diabetes, atrial fibrillation, cardiomyopathy, liver cancer, cirrhosis, dementia, depression, Parkinson's disease, or atopic dermatitis, through quantitative analysis of the PCR product.

In addition, the present invention provides a method of diagnosing diabetes, atrial fibrillation, cardiomyopathy, liver cancer, cirrhosis, dementia, depression, Parkinson's disease, or atopic dermatitis, the method comprising the following steps:
(a) extracting DNAs from vesicles isolated from samples of a normal individual and a subj ect;
(b) performing PCR (Polymerase Chain Reaction) on the extracted DNA using a pair of primers prepared based on a gene sequence present in 16S rDNA to obtain each PCR product; and
(c) determining a case in which a content of vesicles derived from bacteria of the genus *Rothia* is lower than that of the normal individual sample, as diabetes, atrial fibrillation, cardiomyopathy, liver cancer, cirrhosis, dementia, depression, Parkinson's disease, or atopic dermatitis, through quantitative analysis of the PCR product.

As an embodiment of the present invention, the sample in Step (a) may be blood or urine.

As another embodiment of the present invention, the primer pair in Step (b) may be primers of SEQ ID Nos. 1 and 2.

Further, the present invention provides a pharmaceutical composition for preventing or treating one or more diseases selected from the group consisting of diabetes, cardiovascular diseases, liver disease, a cranial nerve disease, and an inflammatory disease, comprising vesicles derived from bacteria of the genus *Rothia* as an active ingredient.

Further, the present invention provides a food composition for preventing or alleviating one or more diseases selected from the group consisting of diabetes, cardiovascular diseases, liver disease, a cranial nerve disease, and an inflammatory disease, comprising vesicles derived from bacteria of the genus *Rothia* as an active ingredient.

Further, the present invention provides an inhalant composition for preventing or treating one or more diseases selected from the group consisting of diabetes, cardiovascular diseases, liver disease, a cranial nerve disease, and an inflammatory disease, comprising vesicles derived from bacteria of the genus *Rothia* as an active ingredient.

Further, the present invention provides a method of preventing or treating one or more diseases selected from the group consisting of diabetes, cardiovascular diseases, liver disease, a cranial nerve disease, and an inflammatory disease, the method comprising a step of administering a pharmaceutical composition comprising vesicles derived from bacteria of the genus *Rothia* as an active ingredient to a subject.

Further, the present invention provides a use of a pharmaceutical composition comprising vesicles derived from bacteria of the genus *Rothia* as an active ingredient for preventing or treating one or more diseases selected from the group consisting of diabetes, cardiovascular diseases, liver disease, a cranial nerve disease, and an inflammatory disease.

Further, the present invention provides a use of vesicles derived from bacteria of the genus *Rothia* for producing a drug used in treating one or more diseases selected from the group consisting of diabetes, cardiovascular diseases, liver disease, a cranial nerve disease, and an inflammatory disease.

As an embodiment of the present invention, the cardiovascular disease may be one or more selected from the group consisting of atrial fibrillation, cardiomyopathy, myocardial infarction, hypertension, ischemic heart disease, coronary artery disease, angina, atherosclerosis, arteriosclerosis, and arrhythmia.

As another embodiment of the present invention, the liver disease may be one or more selected from the group consisting of liver cancer, cirrhosis, hepatitis, and fatty liver.

As another embodiment of the present invention, the cranial nerve disease may be one or more selected from the group consisting of depression, obsessive-compulsive disorder, schizophrenia, dementia, Alzheimer's disease, epilepsy, autism, and Parkinson's disease.

As another embodiment of the present invention, the inflammatory disease may be one or more selected from the group consisting of gingivitis, periodontitis, gastritis, inflammatory enteritis, colitis, atopic dermatitis, acne, hair loss, psoriasis, rhinitis, nasal polyps, asthma, chronic obstructive pulmonary disease (COPD), degenerative arthritis, and rheumatoid arthritis.

Further, the present invention provides a cosmetic composition for preventing or alleviating an inflammatory skin disease, comprising vesicles derived from bacteria of the genus *Rothia* as an active ingredient.

As an embodiment of the present invention, the inflammatory skin disease may be one or more selected from the group consisting of atopic dermatitis, acne, hair loss, and psoriasis.

As an embodiment of the present invention, the vesicles may have an average diameter of 10 to 200 nm.

As another embodiment of the present invention, the vesicles may be secreted naturally or artificially from bacteria of the genus *Rothia.*

As another embodiment of the present invention, the vesicles derived from bacteria of the genus *Rothia* may be secreted from *Rothia amarae.*

### [Advantageous Effects]

The inventors confirmed that intestinal bacteria are not absorbed into the body through epithelial cells, but bacteria-derived vesicles are absorbed, systemically distributed and then excreted out of the body through the kidneys, liver and lungs, and by metagenomic analysis for vesicles derived from bacteria present in patients' blood, also confirmed that vesicles derived from bacteria of the genus *Rothia,* which are present in blood or urine of patients with diabetes, atrial fibrillation, cardiomyopathy, liver cancer, cirrhosis, dementia, depression, Parkinson's disease, and atopic dermatitis significantly decrease, compared with a normal individual. In addition, when inflammatory cells were administered in vitro with vesicles in vitro cultured and isolated from *Rothia amarae* which is one bacterial species of the genus *Rothia,* it was observed that the secretion of inflammation mediators caused by pathogenic vesicles is significantly inhibited. In addition, as it is confirmed that the BDNF expression of nerve cells, which is inhibited by a stress hormone, is significantly recovered by the vesicles, the vesicles derived from bacteria of the genus *Rothia* according to the present invention are expected to be effectively used in a method of diagnosing diabetes, atrial fibrillation, cardiomyopathy, liver cancer, cirrhosis, dementia, depression, Parkinson's disease, and atopic dermatitis, and a food, inhalant or pharmaceutical composition for preventing, alleviating, or treating diabetes, cardiovascular diseases, liver disease, a cranial nerve disease, and an inflammatory disease.

### [Description of Drawings]

FIG. 1A is a series of photographs capturing distribution patterns of bacteria and bacteria-derived vesicles (EV) by time after the bacteria and the vesicles derived from bacteria were orally administered to mice, and FIG. 1B is a result of evaluating the in vivo distribution patterns of the bacteria and the vesicles by harvesting blood, kidneys, liver, and various organs at 12 hours after orally administering the bacteria and the vesicles.
FIG. 2 is a result of comparing the distributions of vesicles derived from bacteria of the genus *Rothia* after metagenomic analysis of bacteria-derived vesicles present in the blood of diabetes patients and a normal individual.
FIG. 3 is a result of comparing the distributions of vesicles derived from bacteria of the genus *Rothia* after metagenomic analysis of bacteria-derived vesicles present in the blood of atrial fibrillation patients and a normal individual.
FIG. 4 is a result of comparing the distributions of vesicles derived from bacteria of the genus *Rothia* after metagenomic analysis of bacteria-derived vesicles present in the blood of cardiomyopathy patients and a normal individual.
FIG. 5 is a result of comparing the distributions of vesicles derived from bacteria of the genus *Rothia* after metagenomic analysis of bacteria-derived vesicles present in the blood of liver cancer patients and a normal individual.
FIG. 6 is a result of comparing the distributions of vesicles derived from bacteria of the genus *Rothia* after metagenomic analysis of bacteria-derived vesicles present in the blood of cirrhosis patients and a normal individual.
FIG. 7 is a result of comparing the distributions of vesicles derived from bacteria of the genus *Rothia* after metagenomic analysis of bacteria-derived vesicles present in the blood of dementia patients and a normal individual.
FIG. 8 is a result of comparing the distributions of vesicles derived from bacteria of the genus *Rothia* after metagenomic analysis of bacteria-derived vesicles present in the blood of depression patients and a normal individual.
FIG. 9 is a result of comparing the distributions of vesicles derived from bacteria of the genus *Rothia* after metagenomic analysis of bacteria-derived vesicles present in the urine of Parkinson's disease patients and a normal individual.
FIG. 10 is a result of comparing the distributions of vesicles derived from bacteria of the genus *Rothia* after metagenomic analysis of bacteria-derived vesicles present in the blood of atopic dermatitis patients and a normal individual.
FIG. 11A is a result obtained by evaluating the influence on secretion of IL-6, which is an inflammation mediator, by *E. coli* vesicles by pretreating vesicles derived from *Rothia* bacteria before treatment of *E. coli* vesicles *(E. coli* EV), which are pathogenic vesicles, to evaluate anti-inflammatory and immunoregulatory effects of *Rothia amarae-derived* vesicles (NC: negative control; PC: positive control, *E. coli* EV 1.0 µg/ml; LP_1.0: *Lactobacillus plantarum* EV 1.0 µg/ml; RAM101: *Rothia amarae* EV).
FIG. 11B is a result obtained by evaluating the influence on secretion of TNF-α, which is an inflammation mediator, by *E. coli* vesicles by pretreating vesicles derived from *Rothia* bacteria before treatment of *E. coli* vesicles *(E. coli* EV), which are pathogenic vesicles, to evaluate anti-inflammatory and immunoregulatory effects of *Rothia amarae-derived* vesicles (NC: negative control; PC: positive control, *E. coli* EV 1.0 µg/ml; LP_1.0: *Lactobacillus plantarum* EV 1.0 µg/ml; RAM101: *Rothia amarae* EV).
FIG. 12 shows the result of evaluating the influence on brain-derived neurotrophic factor (BDNF) expression by nerve cells by simultaneously treating nerve cells, which have been treated with an adrenocortical hormone (GC), which is a stress hormone, with *Rothia amarae-derived* vesicles to evaluate a nerve cell protective effect of the *Rothia amarae-derived* vesicles (EV: *Rothia amarae* extracellular vesicle).

### [Best Modes]

The present invention relates to vesicles derived from bacteria of the genus *Rothia* and a use thereof.

The inventors confirmed that a content of vesicles derived from bacteria of the genus *Rothia* are significantly reduced in samples of patients with diabetes, atrial fibrillation, cardiomyopathy, liver cancer, cirrhosis, dementia, depression, Parkinson's disease, and atopic dermatitis, compared with a normal individual, through metagenomic analysis, and based on this, the present invention was completed.

Thus, the present invention as described above, the present invention provides a method of providing information for diagnosing diabetes, atrial fibrillation, cardiomyopathy, liver cancer, cirrhosis, dementia, depression, Parkinson's disease, or atopic dermatitis, the method comprising the following steps:
(a) extracting DNAs from vesicles isolated from samples of a normal individual and a subj ect;
(b) performing PCR on the extracted DNA using a pair of primers prepared based on a gene sequence present in 16S rDNA to obtain each PCR product; and
(c) classifying a case in which a content of vesicles derived from bacteria of the genus *Rothia* is lower than that of the normal individual sample, as diabetes, atrial fibrillation, cardiomyopathy, liver cancer, cirrhosis, dementia, depression, Parkinson's disease, or atopic dermatitis, through quantitative analysis of the PCR product.

The term "diagnosis" as used herein refers to determination of a condition of a disease of a patient over all aspects, in a broad sense. The contents of the determination are the disease entity, the etiology, the pathogenesis, the severity, the detailed aspects of a disease, the presence and absence of complications, the prognosis, and the like. The diagnosis in the present invention means determining whether diabetes, atrial fibrillation, cardiomyopathy, liver cancer, cirrhosis, dementia, depression, Parkinson's disease, and/or atopic dermatitis occur, the level of the disease, and the like.

The term "nanovesicle" or "vesicle" as used herein refers to a structure consisting of a nano-sized membrane secreted from various bacteria. Vesicles derived from gram-negative bacteria or outer membrane vesicles (OMVs) have endotoxins (lipopolysaccharides), toxic protein, and bacterial DNA and RNA, and vesicles derived from gram-positive bacteria also have peptidoglycan and lipoteichoic acid which are cell wall components of bacteria in addition to proteins and nucleic acids. In the present invention, nanovesicles or vesicles are secreted naturally from bacteria of the genus *Rothia* or produced artificially, are in the form of a sphere, and have an average diameter of 10 to 200 nm.

The term "metagenome" as used herein also refers to a microbiome, and refers to a total of genomes including all viruses, bacteria, fungi, and the like in an isolated region such as soil and an animal's intestines, and is typically used as a concept of genomes explaining identification of a large number of microorganisms at one time by using a sequence analyzer in order to analyze uncultivated microorganisms. In particular, the metagenome does not refer to a genome of one species, but refers to a kind of mixed genome as a genome of all species of one environmental unit. The metagenome is, when one species is defined in the development process of omics biology, a term derived from the viewpoint of making a complete species is made by various species interacting with each other as well as one kind of functionally existing species. Technically, the metagenome is an object of a technique to identify all species in one environment and investigate interactions and metabolism by analyzing all DNAs and RNAs regardless of species using a rapid sequence analysis method.

In the present invention, the sample derived from patients may be blood or urine, but is not limited thereto.

In the present invention, the primer pair in Step (b) may be primers of SEQ ID Nos. 1 and 2, but is not limited thereto.

Another aspect of the present invention provides a composition for preventing or treating one or more diseases selected from the group consisting of diabetes, cardiovascular diseases, liver disease, a cranial nerve disease, and an inflammatory disease, comprising vesicles derived from bacteria of the genus *Rothia* as an active ingredient.

The composition includes a pharmaceutical composition and an inhalant composition.

Further, the present invention provides a method of preventing or treating one or more diseases selected from the group consisting of diabetes, cardiovascular diseases, liver disease, a cranial nerve disease, and an inflammatory disease, the method comprising a step of administering a pharmaceutical composition comprising vesicles derived from bacteria of the genus *Rothia* as an active ingredient to a subject.

Further, the present invention provides a use of a pharmaceutical composition comprising vesicles derived from bacteria of the genus *Rothia* as an active ingredient for preventing or treating one or more diseases selected from the group consisting of diabetes, cardiovascular diseases, liver disease, a cranial nerve disease, and an inflammatory disease.

Further, the present invention provides a use of vesicles derived from bacteria of the genus *Rothia* for producing a drug used in treating one or more diseases selected from the group consisting of diabetes, cardiovascular diseases, liver disease, a cranial nerve disease, and an inflammatory disease.

The term "prevention" as used herein refers to all actions that suppress diabetes, cardiovascular diseases, liver disease, a cranial nerve disease, an inflammatory disease, or the like, or delay the onset thereof via administration of the composition according to the present invention.

The term "treatment" as used herein refers to all actions that alleviate or beneficially change symptoms of diabetes, cardiovascular diseases, liver disease, a cranial nerve disease, an inflammatory disease, or the like via administration of composition according to the present invention.

The term "alleviation" used as used herein refers to all actions that reduce a parameter associated with diabetes, cardiovascular diseases, liver disease, a cranial nerve disease, an inflammatory disease, or the like, for example, the degree of symptoms via administration of composition according to the present invention.

The term "subject" used herein refers to a target in need of treatment of diseases, and more specifically, a mammal such as a human or a non-human primate, a mouse, a rat, a dog, a cat, a horse, a cow, and the like.

The term "administration" used herein means providing a composition of the present invention to a subject by any suitable method.

The term "cardiovascular disease" used herein is a generic term for diseases occurring in the heart and the major arteries, and in the present invention, the cardiovascular disease may be one or more selected from the group consisting of atrial fibrillation, cardiomyopathy, myocardial infarction, hypertension, ischemic heart disease, coronary artery disease, angina, atherosclerosis, arteriosclerosis, and arrhythmia, but the present invention is not limited thereto.

The term "liver disease" used herein is a generic term for impaired liver function, and in the present invention, the liver disease may be one or more selected from the group consisting of liver cancer, cirrhosis, hepatitis, and fatty liver, but is not limited thereto.

The term "cranial nerve disease" used herein is a generic term for diseases caused by problems with cranial nerve cells, and in the present invention, the cranial nerve disease may be one or more selected from the group consisting of depression, obsessive-compulsive disorder, schizophrenia, dementia, Alzheimer's disease, epilepsy, autism and Parkinson's disease, but the present invention is not limited thereto.

The term "inflammatory disease" used herein refers to a disease caused by a chain of biological reactions occurring by a direct reaction of a humoral mediator constituting the immune system or stimulation of a local or systemic effector system, and in the present invention, the inflammatory disease may be one or more selected from the group consisting of gingivitis, periodontitis, gastritis, inflammatory enteritis, colitis, atopic dermatitis, acne, hair loss, psoriasis, rhinitis, nasal polyps, asthma, chronic obstructive pulmonary disease (COPD), degenerative arthritis, and rheumatoid arthritis, but the present invention is not limited thereto.

The vesicles may be isolated from a culturing solution comprising bacteria of the genus *Rothia* by using one or more methods selected from the group consisting of centrifugation, ultra-high speed centrifugation, high pressure treatment, extrusion, sonication, cell lysis, homogenization, freezing-thawing, electroporation, mechanical decomposition, chemical treatment, filtration by a filter, gel filtration chromatography, free-flow electrophoresis, and capillary electrophoresis. Further, a process such as washing for removing impurities and concentration of obtained vesicles may be further included.

In one embodiment of the present invention, as a result of orally administering bacteria and bacteria-derived vesicles to mice and observing in vivo absorption, distribution, and excretion patterns of the bacteria and the vesicles, it was confirmed that, while the bacteria were not absorbed via the intestinal mucous membrane, the bacteria-derived vesicles were absorbed within 5 minutes after administration and systemically distributed, and excreted via the kidneys, liver, and the like (see Example 1).

In another embodiment of the present invention, a bacterial metagenomic analysis was performed by using vesicles isolated from the blood or urine of normal individuals who were matched in age and sex with patients with diabetes, atrial fibrillation, cardiomyopathy, liver cancer, cirrhosis, dementia, depression, Parkinson's disease, and atopic dermatitis. As a result, it was confirmed that vesicles derived from bacteria of the genus *Rothia* were significantly decreased in clinical samples of patients with diabetes, atrial fibrillation, cardiomyopathy, liver cancer, cirrhosis, dementia, depression, Parkinson's disease, and atopic dermatitis as compared to samples of normal individuals (see Examples 3 to 11).

In still another embodiment of the present invention, it was evaluated whether vesicles secreted from the cultured *Rothia amarae* strain exhibit immunoregulatory and anti-inflammatory effects, and as a result of evaluating the secretion of an inflammation mediator by treating macrophages with various concentrations of the *Rothia amarae-derived* vesicles, and then with *E*. coli-derived vesicles, which is an inflammatory disease-causing factor, it was confirmed that the secretion of IL-6 and TNF-α caused by E. coli-derived vesicles is efficiently inhibited by the *Rothia amarae-derived* vesicles (see Example 13).

In still another embodiment of the present invention, the nerve cell protective effect of vesicles derived from a *Rothia amarae* strain was evaluated, and as a result of evaluating brain-derived neurotrophic factor (BDNF) expression by simultaneously treating nerve cells, which have been treated with an adrenocortical hormone, which stresses nerve cells, with *Rothia amarae-derived* vesicles, it was confirmed that the *Rothia amarae-derived* vesicles effectively increase the expression of BDNF, which is a mediator protecting against nerve cell damage (see Example 14).

A content of the vesicles derived from bacteria of the genus *Rothia* in the composition of the present invention can be suitably controlled according to a symptom of a disease, the degree of progression of the symptom, and a patient's condition, and for example, the content of the vesicles may be 0.0001 to 99.9 wt%, or 0.001 to 50 wt% based on the total weight of the composition, but the present invention is not limited thereto. The content ratio is a value determined on the basis of a dry weight from which a solvent was removed.

The pharmaceutical composition according to the present invention may further include a suitable carrier, excipient, and diluent which are commonly used in the preparation of pharmaceutical compositions. The excipient may be, for example, one or more selected from the group consisting of a diluent, a binder, a disintegrant, a lubricant, an adsorbent, a humectant, a film-coating material, and a controlled release additive.

The pharmaceutical composition according to the present invention may be used by being formulated, according to commonly used methods, into a form such as powders, granules, sustained-release-type granules, enteric granules, liquids, eye drops, elixirs, emulsions, suspensions, spirits, troches, aromatic water, lemonades, tablets, sustained-release-type tablets, enteric tablets, sublingual tablets, hard capsules, soft capsules, sustained-release-type capsules, enteric capsules, pills, tinctures, soft extracts, dry extracts, fluid extracts, injections, capsules, perfusates, or a preparation for external use, such as plasters, lotions, pastes, sprays, inhalants, patches, sterile injectable solutions, or aerosols. The preparation for external use may have a formulation such as creams, gels, patches, sprays, ointments, plasters, lotions, liniments, pastes, or cataplasmas.

As the carrier, the excipient, and the diluent that may be included in the pharmaceutical composition according to the present invention, lactose, dextrose, sucrose, oligosaccharides, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil may be used.

For formulation, commonly used diluents or excipients such as fillers, thickeners, binders, wetting agents, disintegrants, and surfactants are used.

As additives of tablets, powders, granules, capsules, pills, and troches according to the present invention, excipients such as corn starch, potato starch, wheat starch, lactose, white sugar, glucose, fructose, D-mannitol, precipitated calcium carbonate, synthetic aluminum silicate, dibasic calcium phosphate, calcium sulfate, sodium chloride, sodium hydrogen carbonate, purified lanolin, microcrystalline cellulose, dextrin, sodium alginate, methyl cellulose, sodium carboxymethylcellulose, kaolin, urea, colloidal silica gel, hydroxypropyl starch, hydroxypropyl methylcellulose 1928, 2208, 2906, 2910, propylene glycol, casein, calcium lactate, and *Primojel*^{®}; and binders such as gelatin, Arabic gum, ethanol, agar powder, cellulose acetate phthalate, carboxymethylcellulose, calcium carboxymethylcellulose, glucose, purified water, sodium caseinate, glycerin, stearic acid, sodium carboxymethylcellulose, sodium methylcellulose, methylcellulose, microcrystalline cellulose, dextrin, hydroxycellulose, hydroxypropyl starch, hydroxymethylcellulose, purified shellac, starch, hydroxypropyl cellulose, hydroxypropyl methylcellulose, polyvinyl alcohol, and polyvinylpyrrolidone may be used, and disintegrants such as hydroxypropyl methylcellulose, corn starch, agar powder, methylcellulose, bentonite, hydroxypropyl starch, sodium carboxymethylcellulose, sodium alginate, calcium carboxymethylcellulose, calcium citrate, sodium lauryl sulfate, silicic anhydride, 1-hydroxypropylcellulose, dextran, ion-exchange resin, polyvinyl acetate, formaldehyde-treated casein and gelatin, alginic acid, amylose, guar gum, sodium bicarbonate, polyvinylpyrrolidone, calcium phosphate, gelled starch, Arabic gum, amylopectin, pectin, sodium polyphosphate, ethyl cellulose, white sugar, magnesium aluminum silicate, a disorbitol solution, and light anhydrous silicic acid; and lubricants such as calcium stearate, magnesium stearate, stearic acid, hydrogenated vegetable oil, talc, lycopodium powder, kaolin, Vaseline, sodium stearate, cacao butter, sodium salicylate, magnesium salicylate, polyethylene glycol 4000, 6000, liquid paraffin, hydrogenated soybean oil (Lubri wax), aluminum stearate, zinc stearate, sodium lauryl sulfate, magnesium oxide, Macrogol, synthetic aluminum silicate, silicic anhydride, higher fatty acids, higher alcohols, silicone oil, paraffin oil, polyethylene glycol fatty acid ether, starch, sodium chloride, sodium acetate, sodium oleate, dl-leucine, and light anhydrous silicic acid may be used.

As additives of liquids according to the present invention, water, dilute hydrochloric acid, dilute sulfuric acid, sodium citrate, monostearic acid sucrose, polyoxyethylene sorbitol fatty acid esters (twin esters), polyoxyethylene monoalkyl ethers, lanolin ethers, lanolin esters, acetic acid, hydrochloric acid, ammonia water, ammonium carbonate, potassium hydroxide, sodium hydroxide, prolamine, polyvinylpyrrolidone, ethylcellulose, and sodium carboxymethylcellulose may be used.

In syrups according to the present invention, a white sugar solution, other sugars or sweeteners, and the like may be used, and as necessary, a fragrance, a colorant, a preservative, a stabilizer, a suspending agent, an emulsifier, a viscous agent, or the like may be used.

In emulsions according to the present invention, purified water may be used, and as necessary, an emulsifier, a preservative, a stabilizer, a fragrance, or the like may be used.

In suspensions according to the present invention, suspending agents such as acacia, tragacanth, methylcellulose, carboxymethylcellulose, sodium carboxymethylcellulose, microcrystalline cellulose, sodium alginate, hydroxypropyl methylcellulose 1828, 2906, 2910, and the like may be used, and as necessary, a surfactant, a preservative, a stabilizer, a colorant, and a fragrance may be used.

Injections according to the present invention may include: solvents such as distilled water for injection, a 0.9% sodium chloride solution, Ringer's solution, a dextrose solution, a dextrose+sodium chloride solution, PEG, lactated Ringer's solution, ethanol, propylene glycol, non-volatile oil-sesame oil, cottonseed oil, peanut oil, soybean oil, corn oil, ethyl oleate, isopropyl myristate, and benzene benzoate; cosolvents such as sodium benzoate, sodium salicylate, sodium acetate, urea, urethane, monoethylacetamide, butazolidine, propylene glycol, the Tween series, amide nicotinate, hexamine, and dimethylacetamide; buffers such as weak acids and salts thereof (acetic acid and sodium acetate), weak bases and salts thereof (ammonia and ammonium acetate), organic compounds, proteins, albumin, peptone, and gums; isotonic agents such as sodium chloride; stabilizers such as sodium bisulfite (NaHSO₃) carbon dioxide gas, sodium metabisulfite (Na₂S₂O₃), sodium sulfite (Na₂SO₃), nitrogen gas (N₂), and ethylenediamine tetraacetic acid; sulfating agents such as 0.1% sodium bisulfide, sodium formaldehyde sulfoxylate, thiourea, disodium ethylenediaminetetraacetate, and acetone sodium bisulfite; a pain relief agent such as benzyl alcohol, chlorobutanol, procaine hydrochloride, glucose, and calcium gluconate; and suspending agents such as sodium CMC, sodium alginate, Tween 80, and aluminum monostearate.

In suppositories according to the present invention, bases such as cacao butter, lanolin, Witepsol, polyethylene glycol, glycerogelatin, methylcellulose, carboxymethylcellulose, a mixture of stearic acid and oleic acid, Subanal, cottonseed oil, peanut oil, palm oil, cacao butter + cholesterol, lecithin, lanette wax, glycerol monostearate, Tween or span, imhausen, monolan(propylene glycol monostearate), glycerin, Adeps solidus, buytyrum Tego-G, cebes Pharma 16, hexalide base 95, cotomar, Hydrokote SP, S-70-XXA, S-70-XX75(S-70-XX95), Hydrokote 25, Hydrokote 711, idropostal, massa estrarium (A, AS, B, C, D, E, I, T), masa-MF, masupol, masupol-15, neosuppostal-N, paramount-B, supposiro OSI, OSIX, A, B, C, D, H, L, suppository base IV types AB, B, A, BC, BBG, E, BGF, C, D, 299, suppostal N, Es, Wecoby W, R, S, M, Fs, and tegester triglyceride matter (TG-95, MA, 57) may be used.

Solid preparations for oral administration include tablets, pills, powders, granules, capsules, and the like, and such solid preparations are formulated by mixing the composition with at least one excipient, e.g., starch, calcium carbonate, sucrose, lactose, gelatin, and the like. In addition to simple excipients, lubricants such as magnesium stearate and talc are also used.

Examples of liquid preparations for oral administration include suspensions, liquids for internal use, emulsions, syrups, and the like, and these liquid preparations may include, in addition to simple commonly used diluents, such as water and liquid paraffin, various types of excipients, for example, a wetting agent, a sweetener, a fragrance, a preservative, and the like. Preparations for parenteral administration include an aqueous sterile solution, a non-aqueous solvent, a suspension, an emulsion, a freeze-dried preparation, and a suppository. Nonlimiting examples of the non-aqueous solvent and the suspension include propylene glycol, polyethylene glycol, a vegetable oil such as olive oil, and an injectable ester such as ethyl oleate.

The pharmaceutical composition according to the present invention is administered in a pharmaceutically effective amount. In the present invention, the pharmaceutically effective amount refers to an amount sufficient to treat diseases at a reasonable benefit/risk ratio applicable to medical treatment, and an effective dosage level may be determined according to factors including types of diseases of patients, the severity of disease, the activity of drugs, sensitivity to drugs, administration time, administration route, excretion rate, treatment period, and simultaneously used drugs, and factors well known in other medical fields.

The composition according to the present invention may be administered as an individual therapeutic agent or in combination with other therapeutic agents, may be administered sequentially or simultaneously with therapeutic agents in the related art, and may be administered in a single dose or multiple doses. It is important to administer the composition in a minimum amount that can obtain the maximum effect without any side effects, in consideration of all the aforementioned factors, and this may be easily determined by those of ordinary skill in the art.

The pharmaceutical composition of the present invention may be administered to an individual via various routes. All administration methods can be predicted, and the pharmaceutical composition may be administered via, for example, oral administration, subcutaneous injection, intraperitoneal administration, intravenous injection, intramuscular injection, intrathecal (space around the spinal cord) injection, sublingual administration, administration via the buccal mucosa, intrarectal insertion, intravaginal insertion, ocular administration, intra-aural administration, intranasal administration, inhalation, spraying via the mouth or nose, transdermal administration, percutaneous administration, or the like.

The pharmaceutical composition of the present invention is determined depending on the type of a drug, which is an active ingredient, along with various related factors such as a disease to be treated, administration route, the age, gender, and body weight of a patient, and the severity of diseases.

The inhalant composition of the present invention may include not only vesicles derived from bacteria of the genus *Rothia,* but also components conventionally used in inhalant compositions, for example, conventional adjuvants such as an antioxidant, a stabilizer, a solubilizer, a vitamin and a flavoring agent, and a carrier.

Another aspect of the present invention provides a food composition for preventing or alleviating one or more diseases selected from the group consisting of diabetes, cardiovascular diseases, liver disease, a cranial nerve disease, and an inflammatory disease, comprising vesicles derived from bacteria of the genus *Rothia* as an active ingredient.

The food composition of the present invention includes a health functional food composition.

When the vesicles derived from bacteria of the genus *Rothia* of the present invention are used as a food additive, the vesicles derived from bacteria of the genus *Rothia* may be added alone or in combination with a different food or food component, or may be suitably used according to a conventional method. The mixed amount of the active ingredient may be suitably determined depending on the purpose of use thereof (for prevention, health, or therapeutic treatment). In general, when a food or beverage is prepared, the vesicles derived from bacteria of the genus *Rothia* of the present invention is added in an amount of 15 wt% or less, preferably 10 wt% or less based on the raw materials. However, in the case of long-term intake for the purpose of health and hygiene or health control, the amount may be less than the above range, and since there is no problem in terms of safety, the active ingredient may be used in an amount above the above range.

There is no particular limitation to the type of food. Examples of the food to which the material can be added may include meat, sausage, bread, chocolate, candy, snacks, confectionaries, pizza, ramen, other noodles, gum, dairy products including ice cream, various types of soups, beverages, tea, drinks, alcoholic beverages and vitamin complexes, and in a general sense, include all health functional foods.

The health beverage composition according to the present invention may contain several favoring agents or natural carbohydrates as additional components, like a conventional beverage. The above-described natural carbohydrates include monosaccharides such as glucose and fructose, disaccharides such as maltose and sucrose, polysaccharides such as dextrin and cyclodextrin, and sugar alcohols such as xylitol, sorbitol and erythritol. As the sweeteners, natural sweeteners such as thaumatin and a stevia extract, and synthetic sweeteners such as saccharin and aspartame, may be used. The proportion of the natural carbohydrate is generally approximately 0.01 to 0.20 g, or 0.04 to 0.10 g per 100 mL of the composition of the present invention.

In addition, the composition of the present invention may contain various nutrients, vitamins, electrolytes, flavoring agents, pectic acid and a salt thereof, alginic acid and a salt thereof, organic acids, protective colloidal thickening agents, pH adjustors, stabilizers, preservatives, glycerin, alcohols, or carbonizing agents used in soda. Other than these, the composition of the present invention may contain fruit pulp for producing natural fruit juices, fruit drinks and vegetable drinks. These components may be used independently or in combination. The proportion of the additive is, but not greatly important, generally selected within the range of 0.01 to 0.20 parts by weight based on 100 parts by weight of the composition of the present invention.

Another aspect of the present invention provides a cosmetic composition for preventing or alleviating an inflammatory skin disease, comprising vesicles derived from bacteria of the genus *Rothia* as an active ingredient.

In the present invention, the inflammatory skin disease may be one or more selected from the group consisting of atopic dermatitis, acne, hair loss, and psoriasis, but is not limited thereto.

A formulation for the cosmetic composition according to the present invention may include a skin lotion, a skin softener, a skin toner, an astringent, a lotion, a milk lotion, a moisturizing lotion, a nourishing lotion, a massage cream, a nourishing cream, a mist, a moisturizing cream, a hand cream, a hand lotion, a foundation, an essence, a nourishing essence, a pack, soap, a cleansing foam, a cleansing lotion, a cleansing cream, a cleansing oil, a cleansing balm, a body lotion or a body cleanser.

A cosmetic composition of the present invention may further include a composition selected from the group consisting of water-soluble vitamins, oil-soluble vitamins, polymer peptides, polymeric polysaccharides, and sphingolipids.

The water-soluble vitamin may be any substance that is blendable with cosmetics, but examples thereof include vitamin B1, vitamin B2, vitamin B6, pyridoxine, pyridoxine hydrochloride, vitamin B12, pantothenic acid, nicotinic acid, nicotinic acid amide, folic acid, vitamin C, vitamin H, and the like, and salts thereof (thiamine hydrochloride, sodium ascorbate, and the like) or derivatives thereof (sodium ascorbic acid-2-phosphate, magnesium ascorbic acid-2-phosphate, and the like) are also included in water-soluble vitamins that may be used in the present invention. These water-soluble vitamins may be obtained by a conventional method such as microbial transformation, purification from a microbial culture, an enzyme method, or a chemical synthesis method.

The oil-soluble vitamins may be any substance that is blendable with cosmetics, but examples thereof include vitamin A, carotene, vitamin D2, vitamin D3, vitamin E (d1-α-tocopherol, d-α-tocopherol), or the like, and derivatives thereof (e.g., ascorbyl palmitate, ascorbyl stearate, ascorbyl dipalmitate, d1-α-tocopherol acetate, d1-α-tocopherol nicotinate, vitamin E, DL-pantothenyl alcohol, D-pantothenyl alcohol, pantothenyl ethylether) may also be included in the oil-soluble vitamins used in the present invention. These oil-soluble vitamins may be obtained by a conventional method such as microbial transformation, purification from a microbial culture, or enzymatic or chemical synthesis.

The polymer peptides may be any substance that is blendable with cosmetics, but examples thereof may include collagen, hydrolyzed collagen, gelatin, elastin, hydrolyzed elastin, and keratin. The polymer peptides may be purified and obtained by any conventional method such as purification from a microbial culture, an enzyme method, or a chemical synthesis method, or may generally be used by being purified from natural substances such as the dermis of a pig, a cow, or the like and silk fiber of silkworms.

The polymeric polysaccharides may be any substance that is blendable with cosmetics, and examples thereof may include hydroxyethyl cellulose, xanthan gum, sodium hyaluronate, and chondroitin sulfate or salts thereof (sodium salts). For example, chondroitin sulfate or salts thereof may generally be purified from mammals or fish and used.

The sphingolipids may be any substance that is blendable with cosmetics, and examples thereof may include ceramide, phytosphingosine, and sphingoglycolipid. The sphingolipids may be purified, by a conventional method, from mammals, fish, shellfish, yeast, or plants, or may be obtained by a chemical synthesis method.

The cosmetic composition of the present invention may include, as necessary, other ingredients mixed in conventional cosmetics along with the above essential ingredients.

Examples of additional ingredients to be mixed may include lipid components, a humectant, an emollient, a surfactant, organic and inorganic pigments, organic powder, a UV absorbent, a preservative, a sanitizer, an antioxidant, a plant extract, a pH adjuster, alcohol, pigments, flavors, a blood circulation promoter, a cooling agent, an anti-diaphoretic, and purified water.

The lipid components may include, for example, ester lipids, hydrocarbon lipids, silicone lipids, fluorine lipids, animal fats, vegetable oil, or the like.

The ester lipids may include, for example, glyceryl tri 2-ethylhexanoate, cetyl 2-ethylhexanoate, isopropyl myristate, butyl myristate, isopropyl palmitate, ethyl stearate, octyl palmitate, isocetyl isostearate, butyl stearate, ethyl linolate, isopropyl linolate, ethyl oleate, isocetyl myristate, isostearyl myristate, isostearyl palmitate, octyldodecyl myristate, isocetyl isostearate, diethyl sebacate, diisopropyl adipate, isoalkyl neopentanate, tri(capryl, capric acid)glyceryl, trimethylolpropane tri 2-ethylhexanoate, trimethylolpropane triisostearate, pentaerythritol tetra 2-ethylhexanoate, cetyl caprylate, decyl laurate, hexyl laurate, decyl myristate, myristyl myristate, cetyl myristate, stearyl stearate, decyl oleate, cetyl ricinoleate, isostearyl laurate, isotridecyl myristate, isocetyl palmitate, octyl stearate, isocetyl stearate, isodecyl oleate, octyldodecyl oleate, octyldodecyl linolate, isopropyl isostearate, cetostearyl 2-ethylhexanoate, stearyl 2-ethylhexanoate, hexyl isostearate, ethyleneglycol dioctanoate, ethyleneglycol dioleate, propyleneglycol dicaprinate, propyleneglycol di(caprylate, caprinate), propyleneglycol dicaprylate, neopentylglycol dicaprinate, neopentylglycol dioctanoate, glyceryl tricaprylate, glyceryl triundecylate, glyceryl triisopalmitate, glyceryl triisostearate, octyldodecyl neopentanoate, isostearyl octanoate, octyl isononanoate, hexyldecyl neodecanoate, octyldodecyl neodecanoate, isocetyl isostearate, isostearyl isostearate, octyldecyl isostearate, polyglycerin ester oleate, polyglycerin ester isostearate, triisocetyl citrate, triisoalkyl citrate, triisooctyl citrate, lauryl lactate, myristyl lactate, cetyl lactate, octyldecyl lactate, triethyl citrate, acetyltriethyl citrate, acetyltributyl citrate, trioctyl citrate, diisostearyl malate, 2-ethylhexyl hydroxystearate, di 2-ethylhexyl succinate, diisobutyl adipate, diisopropyl sebacate, dioctyl sebacate, cholesteryl stearate, cholesteryl isostearate, cholesteryl hydroxystearate, cholesteryl oleate, dihydrocholesteryl oleate, phytosteryl isostearate, phytosteryl oleate, isocetyl 12-stearoyl hydroxystearate, stearoyl 12-stearoyl hydroxystearate, isostearyl 12-stearoyl hydroxystearate, and the like.

The hydrocarbon lipids may include, for example, squalene, liquid paraffin, alphaolefin oligomers, isoparaffin, ceresine, paraffin, liquid isoparaffin, polybutene, microcrystalline wax, Vaseline, and the like.

The silicone lipids may include, for example, polymethyl silicon, methylphenyl silicon, methyl cyclopolysiloxane, octamethyl polysiloxane, decamethyl polysiloxane, dodecamethyl cyclosiloxane, dimethylsiloxane/methylcetyloxysiloxane copolymers, dimethylsiloxane/methylstearoxysiloxane copolymers, alkyl-modified silicon oil, amino-modified silicon oil, and the like.

The fluorine lipids may include perfluoropolyether and the like.

The animal or vegetable oil may include avocado oil, almond oil, olive oil, sesame oil, rice bran oil, safflower oil, soybean oil, corn oil, rape flower oil, apricot kernel oil, palm kernel oil, palm oil, castor oil, sunflower oil, grape seed oil, cotton seed oil, coconut oil, tallow nut oil, wheat germ oil, rice germ oil, Shea butter, evening primrose oil, macadamia nut oil, meadow foam seed oil, yolk oil, beef tallow, hemp seed oil, mink oil, orange roughy oil, jojoba oil, candelilla wax, carnauba wax, liquid lanolin, dehydrated castor oil, and the like.

The humectant may include water-soluble low molecular humectants, oil-soluble molecular humectants, water-soluble polymers, oil-soluble polymers, and the like.

The water-soluble low molecular humectants may include serine, glutamine, sorbitol, mannitol, pyrrolidone-sodium carboxylate, glycerin, propylene glycol, 1,3-butylene glycol, ethylene glycol, polyethylene glycol B (degree of polymerization: n=2 or higher), polypropylene glycol (degree of polymerization: n=2 or higher), polyglycerin B (degree of polymerization: n=2 or higher), lactic acid, lactates, and the like.

The oil-soluble low molecular humectants may include cholesterol, cholesterol ester, and the like.

The water-soluble polymers may include carboxyvinyl polymers, polyasparaginic acid salts, tragacanth, xanthan gum, methyl cellulose, hydroxymethyl cellulose, hydroxylethyl cellulose, hydroxypropyl cellulose, carboxymethyl cellulose, water-soluble chitin, chitosan, dextrin, and the like.

The oil-soluble polymers may include, for example, polyvinyl pyrrolidone/eicosen copolymers, polyvinyl pyrrolidone/hexadecene copolymers, nitrocellulose, dextrin fatty acid ester, silicone polymers, and the like.

The emollients may include, for example, long chain cholesterylester acyl glutamate, cholesteryl hydroxystearate, 12-hydroxystearic acid, stearic acid, rosin acid, lanolin fatty acid cholesteryl ester, and the like.

The surfactants may include, for example, non-ionic surfactants, anionic surfactants, cationic surfactants, amphoteric surfactants, and the like.

The non-ionic surfactants may include self-emulsion type glycerin monostearate, propyleneglycol fatty acid ester, glycerin fatty acid ester, polyglycerin fatty acid ester, sorbitan fatty acid ester, polyoxyethylene (POE) sorbitan fatty acid ester, POE sorbit fatty acid ester, POE glycerin fatty acid ester, POE alkylethers, POE fatty acid ester, POE dehydrated castor oil, POE castor oil, polyoxyethylene/polyoxypropylene (POE/POP) copolymers, POE/POP alkylethers, polyether-modified silicone, alkanolamide laurate, alkylamine oxide, hydrated soy phospholipids, and the like.

The anionic surfactants may include fatty acid soap, α-acylsulfonate, alkyl sulfonates, alkylallyl sulfonates, alkylnaphthalene sulfonates, alkyl sulfates, POE alkylether sulfates, alkylamide sulfates, alkyl phosphates, POE alkyl phosphates, alkylamide phosphates, alkyloyl alkyltaurin salts, N-acylamino acid salts, POE alkylether carboxylates, alkyl sulfosuccinates, sodium alkyl sulfoacetates, acylated hydrolyzed collagen peptide salts, perfluoroalkyl ester phosphates, and the like.

The cationic surfactants may include, for example, alkyltrimethylammonium chloride, stearyltrimethylammonium chloride, steraryltrimethylammonium bromide, cetostearyl trimethylammonium chloride, distearyl dimethylammonium chloride, stearylaryl dimethylbenzylammonium chloride, behenyltrimethylammonium bromide, benzalkonium chloride, diethylaminoethylamide stearate, dimethylaminopropylamide stearate, quaternary ammonium salts of lanolin derivatives, and the like.

The amphoteric surfactants may include carboxybetaine, amidebetaine, sulfobetaine, hydroxysulfobetaine, amidesulfobetaine, phosphobetaine, aminocarboxylate, imidazoline derivatives, amideamine-based amphoteric surfactants, and the like.

The organic and inorganic pigments may include: inorganic pigments such as silicic acid, anhydrous silicic acid, magnesium silicate, talc, sericite, mica, kaolin, bengala, clay, bentonite, titanium dioxide-coated mica, bismuth oxychloride, zirconium oxide, magnesium oxide, zinc oxide, titanium oxide, aluminum oxide, calcium sulfate, barium sulfate, magnesium sulfate, calcium carbonate, magnesium carbonate, iron oxide, ultramarine, chromium oxide, chromium hydroxide, calamine, and combinations thereof; organic pigments such as polyamide, polyester, polypropylene, polystyrene, polyurethane, vinyl resin, urea resin, phenol resin, fluorine resin, silicon resin, acryl resin, melamine resin, epoxy resin, polycarbonate resin, divinyl benzene/styrene copolymers, silk powder, cellulose, CI pigment yellow, and CI pigment orange; and composite pigments of inorganic and organic pigments.

The organic powder may include: metallic soap such as calcium stearate; metal salts of alkyl phosphoric acid such as zinc sodium cetylate, zinc laurylate, and calcium laurylate; polymetallic salts of acylamino acid such as calcium N-lauroyl-beta-alanine, zinc N-lauroyl-beta-alanine, and calcium N-lauroylglycine; polymetallic salts of amide sulfonates such as calcium N-lauroyl-taurine and calcium N-palmitoyl-taurine.; N-acyl alkaline amino acids such as N-epsilon-lauroyl-L-lysine, N-epsilon-palmitoyl lysine, N-α-palmitoylol nitin, N-α-lauroyl arginine, and N-α-dehydrated tallow fatty acid acyl arginine; N-acyl polypeptides such as N-lauroyl glycylglycine; α-amino fatty acids such as α-aminocaprylic acid and α-aminolauric acid; polyethylene; polypropylene; nylon; polymethylmethacrylate; polystyrene; divinylbenzene/styrene copolymers; ethylene tetrafluoride; and the like.

The UV absorbents may include para-aminobenzoic acid, ethyl para-aminobenzoate, amyl para-aminobenzoate, octyl para-aminobenzoate, ethyleneglycol salicylate, phenyl salicylate, octyl salcylate, benzyl salicylate, butylphenyl salicylate, homomentyl salicylate, benzyl cinnamate, para-methoxycinnamic acid-2-ethoxylethyl, octyl paramethoxycinnamate, mono-2-ethylhexaneglyceryl diparamethoxycinnamate, isopropyl paramethoxycinnamate, diisopropyl/diisopropyl cinnamic acid ester mixtures, urocanic acid, ethyl urocanate, hydroxymethoxybenzophenone, hydroxymethoxybenzophenone sulfonic acid and salts thereof, dihydroxymethoxybenzophenone, sodium dihydroxymethoxybenzophenone disulfonate, dihydroxybenzophenone, tetrahydroxybenzophenone, 4-tert-butyl-4'-methoxydibenzoylmethane, 2,4,6-trianilino-p-(carbo-2'-ethylhexyl-1'-oxy)-1,3,5-triazine, 2-(2-hydroxy-5-methylphenyl)benzotriazole, and the like.

The sanitizers may include hinokitiol, trichloric acid, trichlorohydroxydiphenylether, chlorohexidine gluconate, phenoxyethanol, resorcine, isopropylmethylphenol, azulene, salicylic acid, zinc pyrithione, benzalkonium chloride, light sensitive element No. 301, sodium mononitroguaiacol, undecylenic acid, and the like.

The antioxidants may include butylhydroxyanisole, propyl gallate, elisorbic acid, and the like.

The pH adjusters may include citric acid, sodium citrate, malic acid, sodium malate, fumaric acid, sodium fumarate, succinic acid, sodium succinate, sodium hydroxide, sodium monohydrophosphate, and the like.

The alcohols may include higher alcohols such as cetyl alcohol.

In addition, additional ingredients to be mixed are not limited to the above examples, and any one of the above ingredients may be mixed within a range that does not adversely affect the objectives and effects of the present invention, but may range from 0.01 wt% to 5 wt% or 0.01 wt% to 3 wt% with respect to the total weight of the composition.

For lotion, paste, cream, or gel preparations of the present invention, as a carrier ingredient, animal fiber, vegetable fiber, wax, paraffin, starch, tragacanth, a cellulose derivative, polyethylene glycol, silicon, bentonite, silica, talc, zinc oxide, or the like may be used.

For powder or spray preparations of the present invention, lactose, talc, silica, aluminum hydroxide, calcium silicate, or polyamide powder may be used as a carrier ingredient. In particular, in the case of spray preparations, the composition may further include a propellant such as chlorofluorohydrocarbon, propane/butane, or dimethyl ether.

For solution or emulsion preparations of the present invention, a solvent, a solubilizing agent, or an emulsifying agent may be used as a carrier ingredient, and the carrier ingredient may be, for example, water, ethanol, isopropanol, ethyl carbonate, ethyl acetate, benzyl alcohol, benzyl benzoate, propylene glycol, 1,3-butyl glycol oil, a glycerol aliphatic ester, polyethylene glycol, or a sorbitan fatty acid ester.

For suspension preparations of the present invention, as a carrier ingredient, a liquid diluent such as water, ethanol, or propylene glycol, a suspending agent such as ethoxylated isostearyl alcohol, polyoxyethylene sorbitol ester, or polyoxyethylene sorbitan ester, microcrystalline cellulose, aluminum methahydroxide, bentonite, agar, tragacanth, or the like may be used.

For surfactant-containing cleansing preparations of the present invention, as a carrier ingredient, an aliphatic alcohol sulfate, an aliphatic alcohol ether sulfate, a sulfosuccinate monoester, isethionate, imidazolinium derivatives, methyltaurate, sarcosinate, fatty acid amide ether sulfate, alkylamidobetaine, an aliphatic alcohol, a fatty acid glyceride, a fatty acid diethanol amide, vegetable oil, a lanolin derivative, an ethoxylated glycerol fatty acid ester, or the like may be used.

Hereinafter, preferred Examples for helping the understanding of the present invention will be suggested. However, the following Examples are provided only to more easily understand the present invention, and the contents of the present invention are not limited by the following Examples.

### [Examples]

### Example 1. Analysis of in vivo Absorption, Distribution, and Excretion Patterns of Intestinal Bacteria and Vesicles Derived from Bacteria

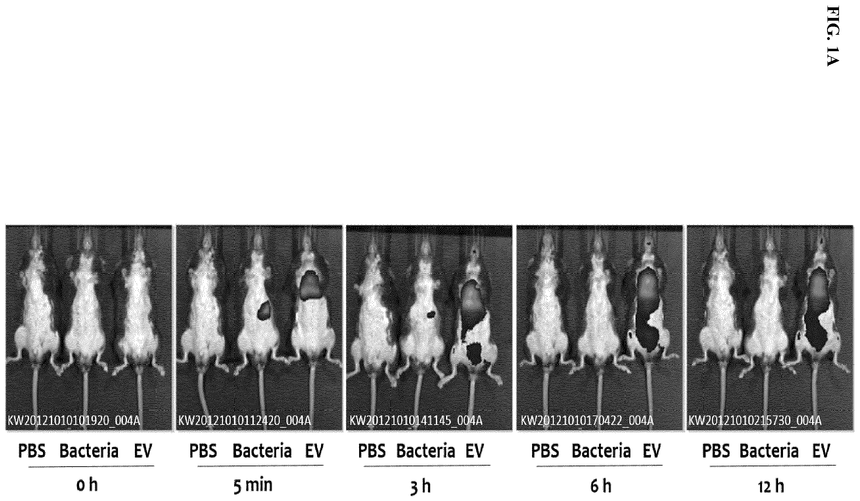

In order to evaluate whether bacteria and bacteria-derived vesicles were systemically absorbed through the mucous, an experiment was performed with the following method. First, a dose of 50 µg of each of fluorescence-labeled intestinal bacteria and the intestinal bacteria-derived vesicles was administered to the stomach of a mouse, and fluorescence was measured after 0 minute, 5 minutes, 3 hours, 6 hours, and 12 hours. As a result of observing the entire image of the mouse, as illustrated in FIG. 1A, the bacteria were not systemically absorbed, but the vesicles derived from bacteria were systemically absorbed 5 minutes after administration, and fluorescence was strongly observed in the bladder 3 hours after administration, so that it could be seen that the vesicles were excreted to the urinary tract. Further, it could be seen that the vesicles were present in the body until 12 hours after administration (see FIG. 1A).

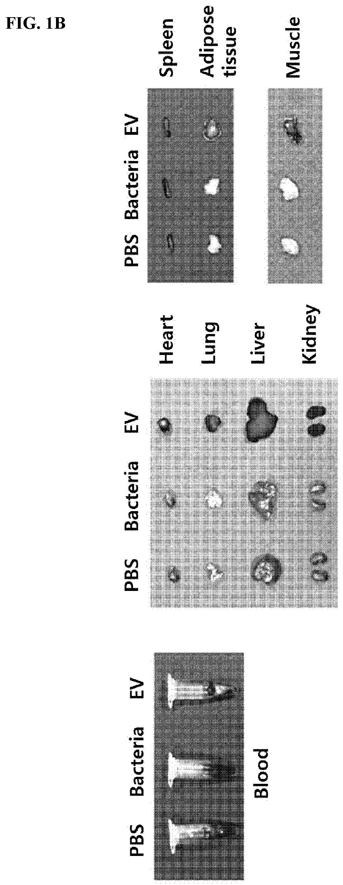

In addition, in order to evaluate the pattern in which the intestinal bacteria and the vesicles derived from the intestinal bacteria infiltrated into various organs after they were systemically absorbed, 50 µg of bacteria and vesicles derived from bacteria labeled with fluorescence were administered in the same manner as described above, and then the urine, heart, lungs, liver, kidneys, spleen, fat, and muscle were collected 12 hours after administration. As a result of observing fluorescence in the collected tissues, as illustrated in FIG. 1B, it could be seen that the vesicles derived from bacteria were distributed in the urine, heart, lungs, liver, spleen, fat, muscle, and kidneys but the bacteria were not absorbed (see FIG. 1B).

### Example 2. Metagenomic Analysis of Vesicles Derived from Bacteria in Clinical Sample

After blood or urine was first put into a 10-ml tube and suspended matter was allowed to settle by a centrifuge (3,500 × g, 10 min, 4°C), only the supernatant was transferred to a new 10-ml tube. After bacteria and impurities were removed by using a 0.22-µm filter, they were transferred to a Centriprep tube (centrifugal filters 50 kD) and centrifuged at 1,500 × g and 4°C for 15 minutes, materials smaller than 50 kD were discarded, and the residue was concentrated to 10 ml. After bacteria and impurities were removed once again by using a 0.22-µm filter, the supernatant was discarded by using a ultra-high speed centrifugation at 150,000 × g and 4°C for 3 hours with a Type 90Ti rotor, and an aggregated pellet was dissolved in physiological saline (PBS).

Internal DNA was extracted out of the lipid by boiling 100 µl of the vesicles isolated by the above method at 100°C, and then cooled on ice for 5 minutes. And then, in order to remove the remaining suspended matter, the DNA was centrifuged at 10,000 × g and 4°C for 30 minutes, and only the supernatant was collected. And, the amount of DNA was quantified by using Nanodrop.

Thereafter, in order to confirm whether the DNA derived from bacteria was present in the extracted DNA, PCR was performed with 16s rDNA primers shown in the following Table 1 and it was confirmed that genes derived from bacteria were present in the extracted genes.

**[Table 1]**

| primer | | Sequence | SEQ ID No. |
|---|---|---|---|
| 16S rDNA | 16S_V3_F | 5'- TCGTCGGCAGCGTCAGATGTGTATAAGA GACAGCCTACGGGNGGCWGCAG-3' | 1 |
| | 16S_V4_R | 5'- GTCTCGTGGGCTCGGAGATGTGTATAAG AGACAGGACTACHVGGGTATCTAATCC | 2 |

The DNA extracted by the above method was amplified using the 16S rDNA primers, and then sequencing was performed (Illumina MiSeq sequencer), the results were output as a standard flowgram format (SFF) file, the SFF file was converted into a sequence file (.fasta) and a nucleotide quality score file using GS FLX software (v2.9), and then the reliability estimation for the reads was confirmed, and a portion in which the window (20 bps) average base call accuracy was less than 99% (Phred score<20) was removed. For the OTU (operational taxonomy unit) analysis, clustering was performed according to sequence similarity by using UCLUST and USEARCH, the genus, family, order, class, and phylum were clustered based on 94%, 90%, 85%, 80%, and 75% sequence similarity, respectively, classification was performed at the phylum, class, order, family, and genus levels of each OUT, and bacteria having a sequence similarity of 97% or more at the genus level were profiled by using the 16S RNA sequence database (108,453 sequences) of BLASTN and GreenGenes (QIIME).

### Example 3. Metagenomic analysis of bacteria-derived vesicles in blood of patient with diabetes

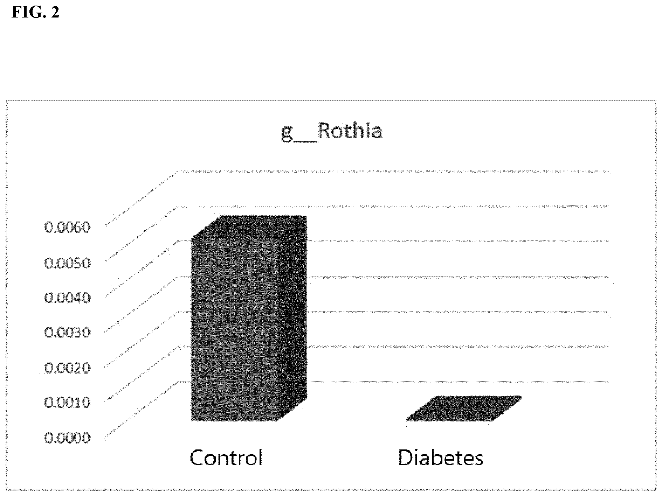

After a metagenomic analysis was performed using the method of Example 2 on the blood from 96 patients with diabetes, and 98 normal individuals who were matched in age and sex by extracting genes from vesicles present in the blood, the distribution of vesicles derived from bacteria of the genus *Rothia* was evaluated. As a result, it was confirmed that vesicles derived from bacteria of the genus *Rothia* were significantly decreased in the blood from the patients with diabetes as compared to the blood from the normal individuals (see Table 2 and FIG. 2).

**[Table 2]**

| Blood | | Control | Diabetes | t-test | | |
|---|---|---|---|---|---|---|
| Taxon | Mean | SD | Mean | SD | p-value | Ratio |
| *Rothia* | 0.0052 | 0.0086 | 0.0001 | 0.0002 | <0.0001 | 0.01 |

### Example 4. Metagenomic analysis of bacteria-derived vesicles in blood of patient with atrial fibrillation

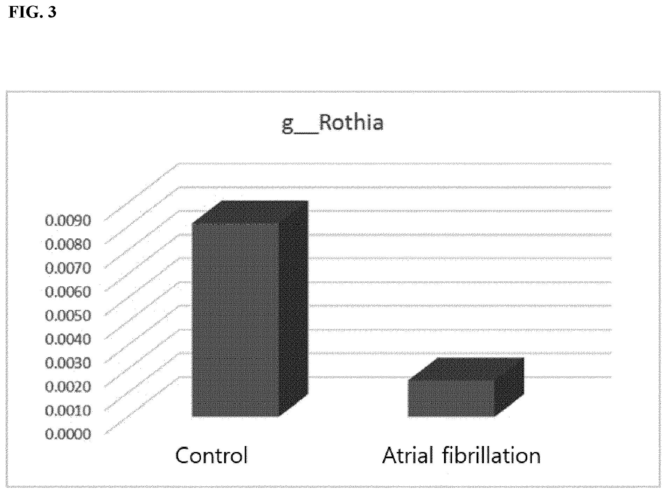

After a metagenomic analysis was performed using the method of Example 2 on the blood from 66 patients with atrial fibrillation, and 71 normal individuals who were matched in age and sex by extracting genes from vesicles present in the blood, the distribution of vesicles derived from bacteria of the genus *Rothia* was evaluated. As a result, it was confirmed that vesicles derived from bacteria of the genus *Rothia* were significantly decreased in the blood from the patients with atrial fibrillation as compared to the blood from the normal individuals (see Table 3 and FIG. 3).

**[Table 3]**

| Blood | Control | | Atrial fibrillation | | t-test | |
|---|---|---|---|---|---|---|
| Taxon | Mean | SD | Mean | SD | p-value | Ratio |
| *Rothia* | 0.0081 | 0.0265 | 0.0015 | 0.0018 | 0.05 | 0.19 |

### Example 5. Metagenomic analysis of bacteria-derived vesicles in blood of patient with cardiomyopathy

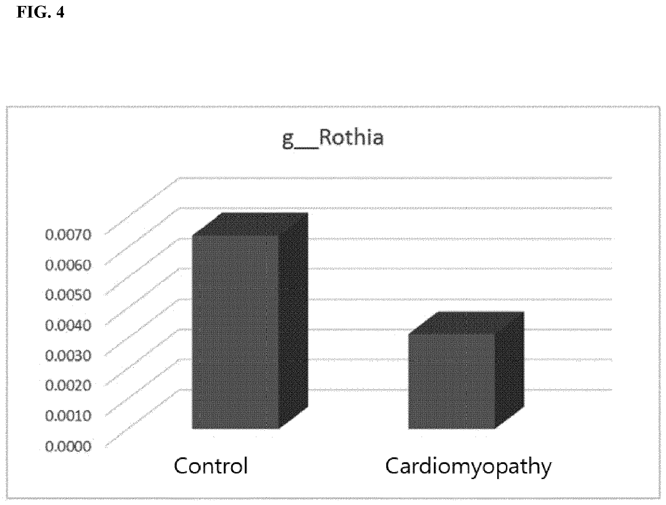

After a metagenomic analysis was performed using the method of Example 2 on the blood from 59 patients with cardiomyopathy, and 69 normal individuals who were matched in age and sex by extracting genes from vesicles present in the blood, the distribution of vesicles derived from bacteria of the genus *Rothia* was evaluated. As a result, it was confirmed that vesicles derived from bacteria of the genus *Rothia* were significantly decreased in the blood from the patients with cardiomyopathy as compared to the blood from the normal individuals (see Table 4 and FIG. 4).

**[Table 4]**

| Blood | Control | | Cardiomyopathy | | t-test | |
|---|---|---|---|---|---|---|
| Taxon | Mean | SD | Mean | SD | p-value | Ratio |
| *Rothia* | 0.0064 | 0.0114 | 0.0031 | 0.0069 | 0.008 | 0.49 |

### Example 6. Metagenomic analysis of bacteria-derived vesicles in blood of patient with liver cancer

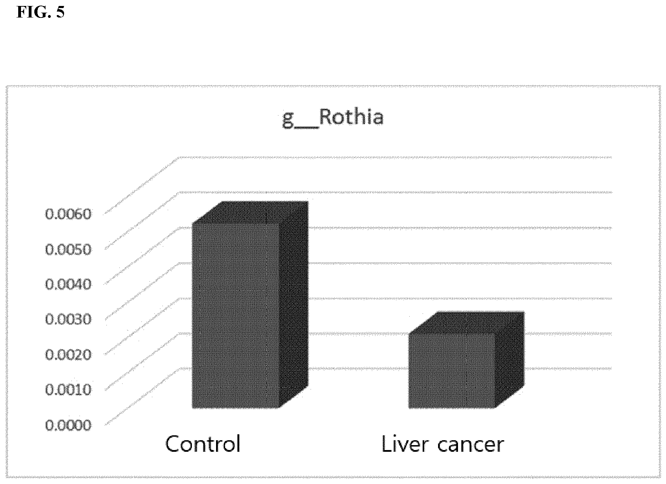

After a metagenomic analysis was performed using the method of Example 2 on the blood from 97 patients with liver cancer, and 109 normal individuals who were matched in age and sex by extracting genes from vesicles present in the blood, the distribution of vesicles derived from bacteria of the genus *Rothia* was evaluated. As a result, it was confirmed that vesicles derived from bacteria of the genus *Rothia* were significantly decreased in the blood from the patients with liver cancer as compared to the blood from the normal individuals (see Table 5 and FIG. 5).

**[Table 5]**

| Blood | Control | | Liver cancer | | t-test | |
|---|---|---|---|---|---|---|
| Taxon | Mean | SD | Mean | SD | p-value | Ratio |
| *Rothia* | 0.0052 | 0.0109 | 0.0021 | 0.0026 | 0.006 | 0.40 |

### Example 7. Metagenomic analysis of bacteria-derived vesicles in blood of patient with cirrhosis

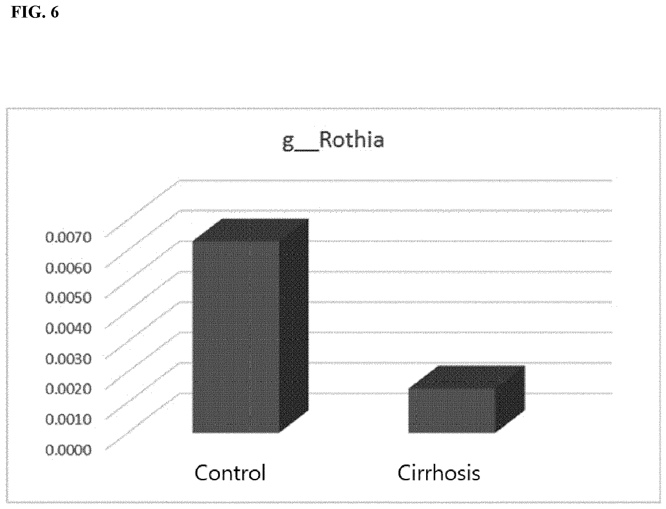

After a metagenomic analysis was performed using the method of Example 2 on the blood from 101 patients with cirrhosis, and 126 normal individuals who were matched in age and sex by extracting genes from vesicles present in the blood, the distribution of vesicles derived from bacteria of the genus *Rothia* was evaluated. As a result, it was confirmed that vesicles derived from bacteria of the genus *Rothia* were significantly decreased in the blood from the patients with cirrhosis as compared to the blood from the normal individuals (see Table 6 and FIG. 6).

**[Table 6]**

| Blood | Control | | Cirrhosis | | t-test | |
|---|---|---|---|---|---|---|
| Taxon | Mean | SD | Mean | SD | p-value | Ratio |
| *Rothia* | 0.0063 | 0.0117 | 0.00015 | 0.0015 | <0.0001 | 0.23 |

### Example 8. Metagenomic analysis of bacteria-derived vesicles in blood of patient with dementia

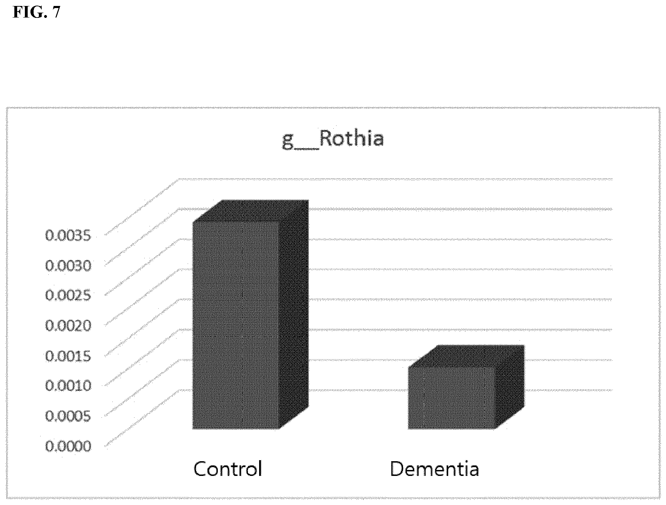

After a metagenomic analysis was performed using the method of Example 2 on the blood from 61 patients with dementia, and 70 normal individuals who were matched in age and sex by extracting genes from vesicles present in the blood, the distribution of vesicles derived from bacteria of the genus *Rothia* was evaluated. As a result, it was confirmed that vesicles derived from bacteria of the genus *Rothia* were significantly decreased in the blood from the patients with dementia as compared to the blood from the normal individuals (see Table 7 and FIG. 7).

**[Table 7]**

| Blood | Control | | Dementia | | t-test | |
|---|---|---|---|---|---|---|
| Taxon | Mean | SD | Mean | SD | p-value | Ratio |
| *Rothia* | 0.0034 | 0.0101 | 0.0010 | 0.0018 | <0.0001 | 0.29 |

### Example 9. Metagenomic analysis of bacteria-derived vesicles in blood of patient with depression

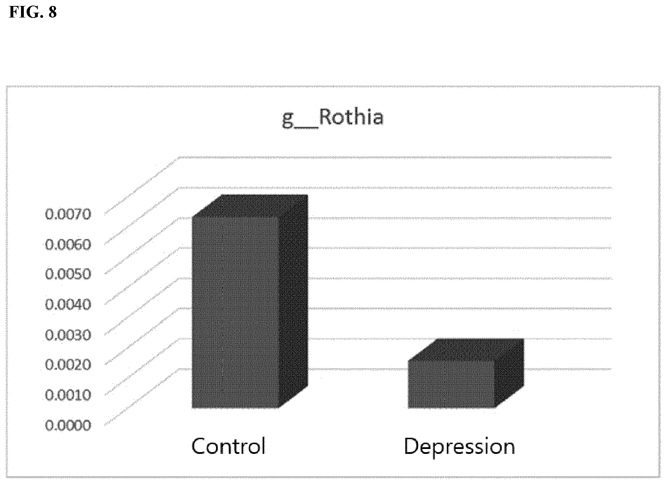

After a metagenomic analysis was performed using the method of Example 2 on the blood from 84 patients with depression, and 92 normal individuals who were matched in age and sex by extracting genes from vesicles present in the blood, the distribution of vesicles derived from bacteria of the genus *Rothia* was evaluated. As a result, it was confirmed that vesicles derived from bacteria of the genus *Rothia* were significantly decreased in the blood from the patients with depression as compared to the blood from the normal individuals (see Table 8 and FIG. 8).

**[Table 8]**

| Blood | Control | | Depression | | t-test | |
|---|---|---|---|---|---|---|
| Taxon | Mean | SD | Mean | SD | p-value | Ratio |
| *Rothia* | 0.0063 | 0.0231 | 0.0016 | 0.0039 | 0.04 | 0.24 |

### Example 10. Metagenomic analysis of bacteria-derived vesicles in urine of patient with Parkinson's disease

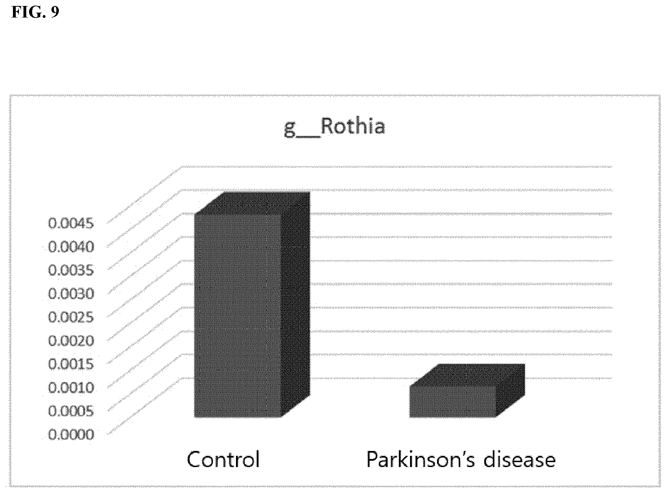

After a metagenomic analysis was performed using the method of Example 2 on the urine from 54 patients with Parkinson's disease, and 62 normal individuals who were matched in age and sex by extracting genes from vesicles present in the urine, the distribution of vesicles derived from bacteria of the genus *Rothia* was evaluated. As a result, it was confirmed that vesicles derived from bacteria of the genus *Rothia* were significantly decreased in the urine from the patients with Parkinson's disease as compared to the urine from the normal individuals (see Table 9 and FIG. 9).

**[Table 9]**

| Urine | Control | | Parkinson's disease | | t-test | |
|---|---|---|---|---|---|---|
| Taxon | Mean | SD | Mean | SD | p-value | Ratio |
| *Rothia* | 0.0043 | 0.0058 | 0.0007 | 0.0014 | <0.0001 | 0.15 |

### Example 11. Metagenomic analysis of bacteria-derived vesicles in blood of patient with atopic dermatitis

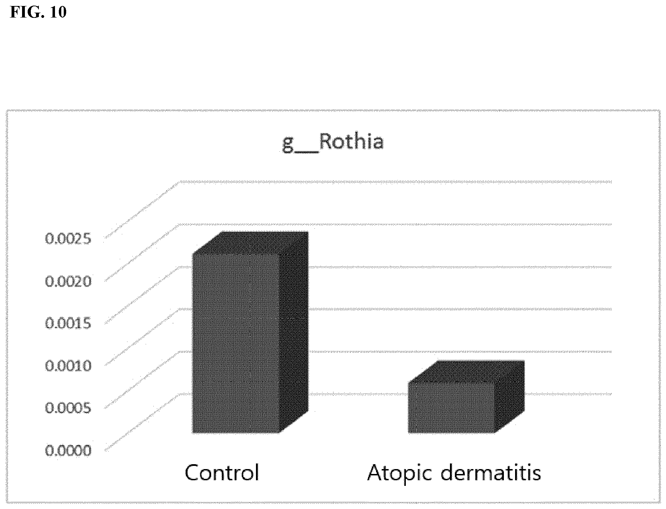

After a metagenomic analysis was performed using the method of Example 2 on the blood from 57 patients with atopic dermatitis, and 63 normal individuals who were matched in age and sex by extracting genes from vesicles present in the blood, the distribution of vesicles derived from bacteria of the genus *Rothia* was evaluated. As a result, it was confirmed that vesicles derived from bacteria of the genus *Rothia* were significantly decreased in the blood from the patients with atopic dermatitis as compared to the blood from the normal individuals (see Table 10 and FIG. 10).

**[Table 10]**

| Blood | Control | | Atopic dermatitis | | t-test | |
|---|---|---|---|---|---|---|
| Taxon | Mean | SD | Mean | SD | p-value | Ratio |
| *Rothia* | 0.0021 | 0.0059 | 0.0006 | 0.0008 | 0.004 | 0.28 |

### Example 12. Isolation of Vesicles derived from Rothia amarae

Based on the result of the above examples, the *Rothia amarae* strain belonging to the genus *Rothia* was cultured and then its vesicles were isolated therefrom. The *Rothia amarae* strain was cultured in a brain heart infusion (BHI) medium in a 37 °C aerobic chamber to an absorbance (OD600) of 1.0 to 1.5, and then sub-cultured. Afterward, the medium supernatant not containing the strain was collected and centrifuged at 10,000 g and 4 °C for 15 minutes, filtered through a 0.45-µm filter, and the filtered supernatant was concentrated to a 200 mL volume through ultrafiltration using a QuixStand benchtop system (GE Healthcare, UK) with a 100 kDa hollow filter membrane. Afterward, the concentrated supernatant was filtered again through a 0.22-µm filter, the filtered supernatant was ultracentrifuged at 150,000 g and 4 °C for 3 hours, the pellet was suspended with Dulbecco's Phosphate Buffered Saline (DPBS). Subsequently, density-gradient centrifugation was performed using 10 %, 40 %, and 50 % OptiPrep solutions (Axis-Shield PoC AS, Norway), and to prepare a low-density solution, the OptiPrep solution was diluted with HEPES-buffered saline (20 mM HEPES, 150 mM NaCl, pH 7.4). Centrifugation was performed at 200,000 g and 4 °C for 2 hours, and each solution fractionated into the same volume of 1 mL from the upper layer was additionally ultracentrifuged at 150,000 g and 4 °C for 3 hours. Afterward, a protein was quantified using a bicinchoninic acid (BCA) assay, and an experiment was performed on the obtained vesicles.

### Example 13. Anti-inflammatory Effects of Vesicles derived from Rothia amarae

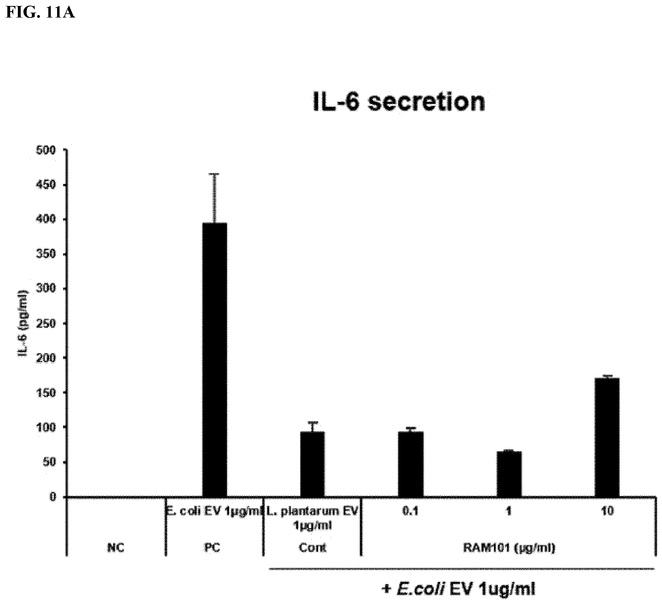

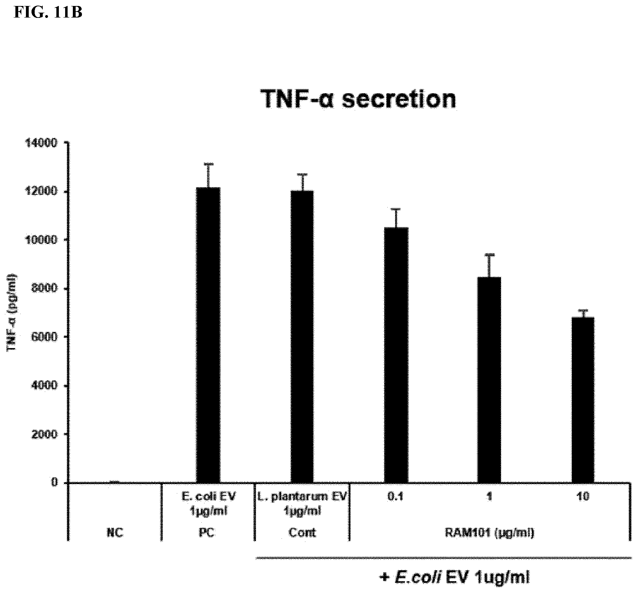

To examine the effect of *Rothia amarae-derived* vesicles on the secretion of inflammation mediators in inflammatory cells, Raw 264.7 cells, which is a mouse macrophage cell line, were treated with the *Rothia amarae-derived* vesicles at various concentrations (0.1, 1 and 10 µg/mL), and then treated with *E. coli* EVs, which are pathogenic vesicles of an inflammatory disease, followed by measuring secretion amounts of the inflammation mediators (IL-6 and TNF-α). More specifically, the Raw 264.7 cells were seeded in a 24-well cell culture plate at 1 × 10⁵ cells/well, and cultured in complete DMEM for 24 hours. Afterward, a culture supernatant was collected in a 1.5 mL tube and centrifuged at 3000 g for 5 minutes, and then the resulting supernatant was stored at 4 °C and subjected to ELISA analysis. As a result, when *Rothia amarae-derived* vesicles were pretreated, it was confirmed that the secretion of the IL-6 (see FIG. 11A) and TNF-α (see FIG. 11B) by factor causing inflammation was significantly suppressed. This result shows that inflammatory responses induced by factor causing inflammation such as *E*. *coli*-derived vesicles can be effectively inhibited by the *Rothia amarae-derived* vesicles.

### Example 14. Nerve cell protective effect of Rothia amarae-derived vesicles

A brain-derived neurotrophic factor (BDNF) is a major mediator protecting nerve cells from nerve cell damage, and its expression is decreased in dementia, depression, Alzheimer's disease, autism, and the like. In this example, to evaluate the therapeutic effect of *Rothia amarae-*derived vesicles against a cranial nerve disease, the nerve cell protective effect was evaluated by treating the nerve cells with a stress hormone. That is, nerve cells (hippocampal neuronal cell line, HT22 cells) were cultured with an adrenocortical hormone (GC: corticosterone 400 ng/ml) or *Rothia amarae-derived* vesicles (EV, 20 µg/mL) for 24 hours in vitro, and the BDNF expression was evaluated by PCR.

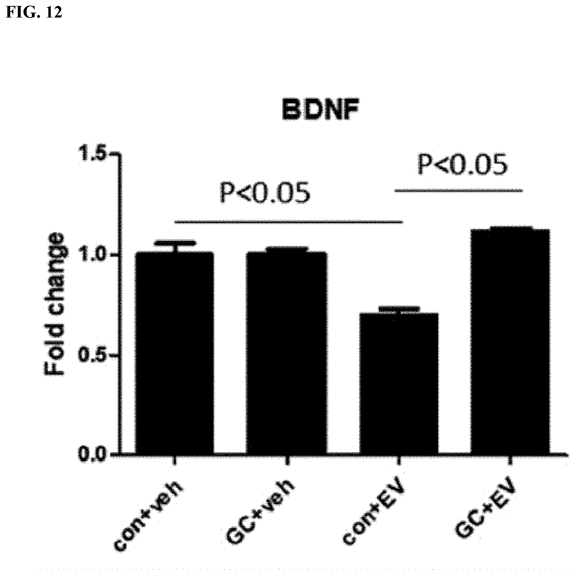

As a result, it was confirmed that the BDNF expression inhibited by adrenocortical hormone treatment is significantly recovered by the treatment of *Rothia amarae-derived* vesicles (see FIG. 12). This means that the *Rothia amarae-derived* vesicles can effectively inhibit a cranial nerve disease caused by a factor causing cranial nerve cell damage such as stress.

The above-described description of the present invention is provided for illustrative purposes, and those of ordinary skill in the art to which the present invention pertains will understand that the present invention can be easily modified into other specific forms without changing the technical spirit or essential features of the present invention. Therefore, it should be understood that the above-described Examples are illustrative only in all aspects and are not restrictive.

### [Industrial Applicability]

Vesicles derived from bacteria of the genus *Rothia* according to the present invention are expected to be effectively used in a method of diagnosing diabetes, atrial fibrillation, cardiomyopathy, liver cancer, cirrhosis, dementia, depression, Parkinson's disease, or atopic dermatitis; and a food, inhalant, cosmetic or pharmaceutical composition for preventing, alleviating or treating diabetes, cardiovascular diseases, liver disease, a cranial nerve disease, or an inflammatory disease.

## Claims

1. A method of providing information for diagnosing diabetes, atrial fibrillation, cardiomyopathy, liver cancer, cirrhosis, dementia, depression, Parkinson's disease, or atopic dermatitis, the method comprising the following steps:
(a) extracting DNAs from vesicles isolated from samples of a normal individual and a subj ect;
(b) performing PCR (Polymerase Chain Reaction) on the extracted DNA using a pair of primers prepared based on a gene sequence present in 16S rDNA to obtain each PCR product; and
(c) classifying a case in which a content of vesicles derived from bacteria of the genus *Rothia* is lower than that of the normal individual sample, as diabetes, atrial fibrillation, cardiomyopathy, liver cancer, cirrhosis, dementia, depression, Parkinson's disease, or atopic dermatitis, through quantitative analysis of the PCR product.

2. The method of claim 1, wherein the sample in Step (a) is blood or urine.

3. A pharmaceutical composition for preventing or treating one or more diseases selected from the group consisting of diabetes, cardiovascular diseases, liver disease, a cranial nerve disease, and an inflammatory disease, comprising vesicles derived from bacteria of the genus *Rothia* as an active ingredient.

4. The pharmaceutical composition of claim 3, wherein the cardiovascular disease is one or more selected from the group consisting of atrial fibrillation, cardiomyopathy, myocardial infarction, hypertension, ischemic heart disease, coronary artery disease, angina, atherosclerosis, arteriosclerosis, and arrhythmia.

5. The pharmaceutical composition of claim 3, wherein the liver disease is one or more selected from the group consisting of liver cancer, cirrhosis, hepatitis, liver cirrhosis, and fatty liver.

6. The pharmaceutical composition of claim 3, wherein the cranial nerve disease is one or more selected from the group consisting of depression, obsessive-compulsive disorder, schizophrenia, dementia, Alzheimer's disease, epilepsy, autism, and Parkinson's disease.

7. The pharmaceutical composition of claim 3, wherein the inflammatory disease is one or more selected from the group consisting of gingivitis, periodontitis, gastritis, inflammatory enteritis, colitis, atopic dermatitis, acne, hair loss, psoriasis, rhinitis, nasal polyps, asthma, chronic obstructive pulmonary disease (COPD), degenerative arthritis, and rheumatoid arthritis.

8. The pharmaceutical composition of claim 3, wherein the vesicles have an average diameter of 10 to 200 nm.

9. The pharmaceutical composition of claim 3, wherein the vesicles are secreted naturally or artificially from bacteria of the genus *Rothia.*

10. The pharmaceutical composition of claim 3, wherein the vesicles derived from bacteria of the genus *Rothia* are secreted from *Rothia amarae.*

11. A food composition for preventing or alleviating one or more diseases selected from the group consisting of diabetes, cardiovascular diseases, liver disease, a cranial nerve disease, and an inflammatory disease, comprising vesicles derived from bacteria of the genus *Rothia* as an active ingredient.

12. The food composition of claim 11, wherein the vesicles have an average diameter of 10 to 200 nm.

13. The food composition of claim 11, wherein the vesicles are secreted naturally or artificially from bacteria of the genus *Rothia.*

14. The food composition of claim 11, wherein the vesicles derived from bacteria of the genus *Rothia* are secreted from *Rothia amarae.*

15. An inhalant composition for preventing or treating one or more diseases selected from the group consisting of diabetes, cardiovascular diseases, liver disease, a cranial nerve disease, and an inflammatory disease, comprising vesicles derived from bacteria of the genus *Rothia* as an active ingredient.

16. A cosmetic composition for preventing or alleviating an inflammatory skin disease, comprising vesicles derived from bacteria of the genus *Rothia* as an active ingredient.

17. The cosmetic composition of claim 16, wherein the inflammatory skin disease is one or more selected from the group consisting of atopic dermatitis, acne, hair loss, and psoriasis.

18. A method of diagnosing diabetes, atrial fibrillation, cardiomyopathy, liver cancer, cirrhosis, dementia, depression, Parkinson's disease, or atopic dermatitis, the method comprising the following steps:
(a) extracting DNAs from vesicles isolated from samples of a normal individual and a subj ect;
(b) performing PCR (Polymerase Chain Reaction) on the extracted DNA using a pair of primers prepared based on a gene sequence present in 16S rDNA to obtain each PCR product; and
(c) determining a case in which a content of vesicles derived from bacteria of the genus *Rothia* is lower than that of the normal individual sample, as diabetes, atrial fibrillation, cardiomyopathy, liver cancer, cirrhosis, dementia, depression, Parkinson's disease, or atopic dermatitis, through quantitative analysis of the PCR product.

19. The method of claim 18, wherein the sample in Step (a) is blood or urine.

20. A method of preventing or treating one or more diseases selected from the group consisting of diabetes, cardiovascular diseases, liver disease, a cranial nerve disease, and an inflammatory disease, the method comprising a step of administering a pharmaceutical composition comprising vesicles derived from bacteria of the genus *Rothia* as an active ingredient to a subject.

21. A use of a pharmaceutical composition comprising vesicles derived from bacteria of the genus *Rothia* as an active ingredient for preventing or treating one or more diseases selected from the group consisting of diabetes, cardiovascular diseases, liver disease, a cranial nerve disease, and an inflammatory disease.

22. A use of vesicles derived from bacteria of the genus *Rothia* for producing a drug used in treating one or more diseases selected from the group consisting of diabetes, cardiovascular diseases, liver disease, a cranial nerve disease, and an inflammatory disease.


<110>	MD Healthcare Inc.

<120>	Nanovesicles derived from Rothia bacteria and Use thereof

<130>	MPCT20-003

<150>	KR 10-2019-0017064
<151>	2019-02-14

<150>	KR 10-2020-0012637
<151>	2020-02-03

<160>	2

<170>	KoPatentIn 3.0

<210>	1
<211>	50
<212>	DNA
<213>	Artificial Sequence

<220>
<223>	16S_V3_F


<220>
<221>	misc_feature
<222>	(42)
<223>	n = a or g or c or t/u


<400>	1
tcgtcggcag cgtcagatgt gtataagaga cagcctacgg gnggcwgcag	50


<210>	2
<211>	55
<212>	DNA
<213>	Artificial Sequence

<220>
<223>	16S_V4_R


<400>	2
gtctcgtggg ctcggagatg tgtataagag acaggactac hvgggtatct aatcc	55